# EUROPEAN PATENT SPECIFICATION

(11) **EP 0 819 250 B1**
(45) Date of publication and mention of the grant of the patent: **20.09.2006**
(21) Application number: 96910404.1
(22) Date of filing: 11.03.1996
(51) Int. Cl.: C12N 5/08, B03C 1/00

(54) **MULTIPARAMETER CELL SEPARATION USING RELEASABLE COLLOIDAL MAGNETIC PARTICLES**
VERFAHREN ZUR FREISETZUNG VON MAGNETISCHEN TEILCHEN, DIE AN ZELLOBERFLÄCHEN GEBUNDEN SIND
SEPARATION CELLULAIRE MULTI-PARAMETRE FAISANT APPEL A DES PARTICULES MAGNETIQUES COLLO DALES LIBERABLES

(30) Priority: 06.04.1995 US 418639
(43) Date of publication of application: 21.01.1998
(73) Proprietor: Miltenyi Biotec Inc., Auburn, CA 94088 (US)
(72) Inventor: MILTENTI, Stephan, D-51429 Bergisch Gladbach (DE)
(74) Representative: Walton, Seán Malcolm
(86) International application number: PCT/US1996/003267
(87) International publication number: WO 1996/031776

(56) References cited:
- EP-A- 0 395 355
- WO-A-90/04019
- WO-A-91/09938
- WO-A-92/18643
- WO-A-94/02016
- US-A- 4 452 773
- US-A- 4 714 680
- DNA AND CELL BIOLOGY, vol. 12, no. 2, 1993, pages 191-197, XP000572336 CHARLOTTA LJUNGQUIST ET AL: "Immobilization and recovery of fusion proteins and B-lymphocyte cells using magnetic separation"

## Description

### INTRODUCTION

### Technical Field

The field of the invention is magnetic cell sorting.

### Background

Multiparameter cell sorting and its benefits are known from the use of fluorescent activated cell sorting (FACS). Cells in suspension can be separated according to as many as four parameters at one time with this technique. While FACS is advantageous for this ability, it has many disadvantages due to the serial nature of the sorting process, where cells must be processed one at a time.

Several methods of parallel sorting have been described. Among them are large magnetic beads, panning, affinity columns, and high gradient magnetic separation (HGMS). Parallel sorting methods provide several benefits when compared to flow cytometry. They only require inexpensive reagents and apparatus, which are easily used and maintained. Many samples can be processed at the same time. An automated system can be used to simplify processing of large sample numbers.

Parallel sorting methods, such as magnetic particle sorting, have the disadvantage that only one parameter can be processed at a time. Cells are either bound or not to a solid support. In practical terms this has limited the available separations to either (a) one step enrichment or depletion; or (b) two step separations where depletion is followed by enrichment. It is only possible to perform double enrichments, or enrichment followed by depletion, if one can effectively remove the magnetic label from the cells.

Proposals for such removal have been put forward. European patent EP0395355 describes the release of magnetic beads from a cell surface by treatment with chymopapain. This has the disadvantage of destroying many other cellular antigens at the same time. International patent application WO 91/15766 describes the use of antibodies to displace antibodies bound to magnetic particles. The procedure is lengthy, and does not work well for many high affinity antibodies.

There is therefore substantial interest in methods for efficiently removing magnetic particles after separation, without affecting the cellular complement of surface antigens.

### Relevant Literature

U.S. Patent 4,452,773 describes the preparation of magnetic iron-dextran microspheres and provides a summary describing the various means of preparation of particles suitable for attachment to biological materials. U.S. Patent no. 4,230,685 describes improvements in attaching specific binding agents to the magnetic particles. A description of polymeric coatings for magnetic particles used in high gradient magnetic sorting, (HGMS) are found in PCT application WO 85/04330. Methods to prepare superparamagnetic particles are described in U.S. Patent 4,770,183. Superparamagnetic materials are highly magnetically susceptible, i.e. they become strongly magnetic when placed in a magnetic field, but rapidly lose their magnetism when removed from the field. This occurs in ferromagnetic materials when the crystal diameter is decreased to a critical value.

European patent EP0395355 describes the release of magnetic beads from a cell surface by treatment with chymopapain. International patent application WO 91/15766 describes the use of antibodies to displace antibodies bound to magnetic particles. U.S. Patent 5,240,640 describes the release of gelatin coated magnetic particles by treatment with collagenase. N. Pope *et al.* (1994) J. Biomed. Materials Res. **28**:449-457 describe the use of alginate as a releasable coating for magnetic particles.

Ljungquist et al DNA & Cell Biology 12 (1993) 191-197 describes the immobilization of fusion proteins and β-lymphocytes linked to a lac repressor sequence to magnetic beads which display the lac operator sequence. Cells and proteins are recovered by various means including DNAse1 digestion.

Schlossman et al J. Immunol 110 (1973) 313 describes the enrichment of lymphocytes using an insoluble Sephadex support which is digested with dextranase.

Basch et al J. Immunol. Methods 56 (1983) 269-280 describes cell separation using positive immunoselective techniques and notes that ligand-coupled magnetic beads have been used to separate cells.

WO94/02016 describes methods for positive immunoselection of stem cells by immobilisation on a solid phase matrix via a hapten/immune agent interaction and elution of the cells using soluble hapten.

### SUMMARY OF THE INVENTION

Methods and compositions are provided for releasing colloidal superparamagnetic particles bound to a cell surface through an affinity reagent.

The particle is released through action of a glycosidase specific for a glycosidic linkage present in at least one of (a) the coating of the particle and (b) a linkage group between the coating and the affinity reagent. The glycosidic linkage is absent in normal mammalian cells. The release process is useful in sequential cell separation processes.

### BRIEF DESCRIPTION OF THE DRAWINGS

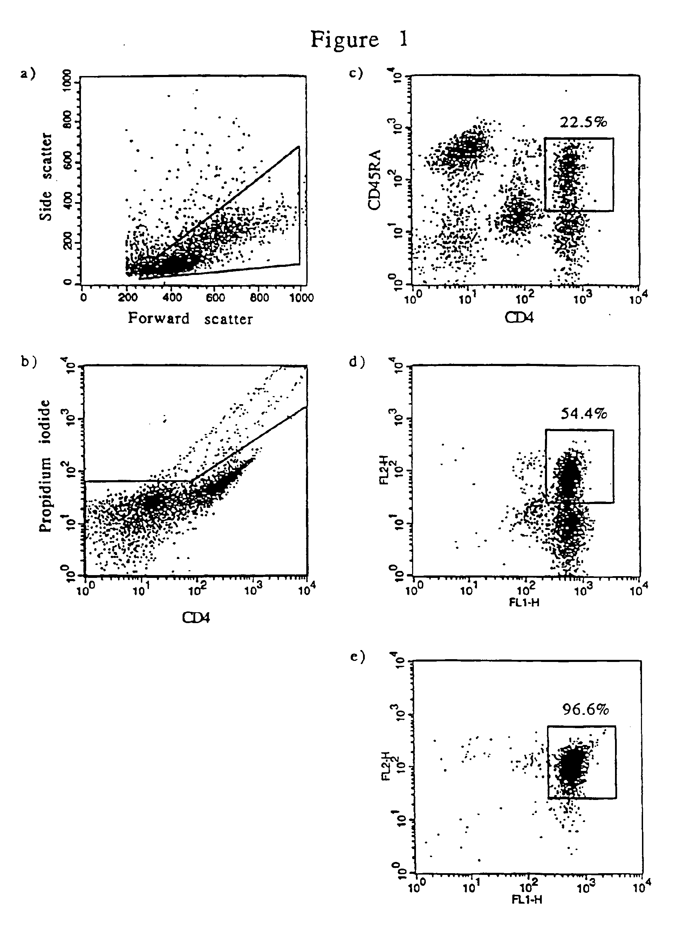

Figure 1 shows an analysis of a magnetic separation of CD45RA expressing T helper cells from PBMC. Figure 1a shows the forward-side scatter gating used for analysis. Figure 1b shows the PI versus CD45RA gating used for analysis. Figure 1c shows a Dot Plot of CD4 (FI-1) versus CD45RA (FI-2) of the cells before separation. Figure 1d shows a Dot Plot of CD4 (FI-1) versus CD45RA (FI-2) of the magnetic fraction after CD4 separation. Figure 1e shows a Dot Plot of CD4 (FI-1) versus CD45RA (FI-2) of the magnetic fraction after CD45RA separation.

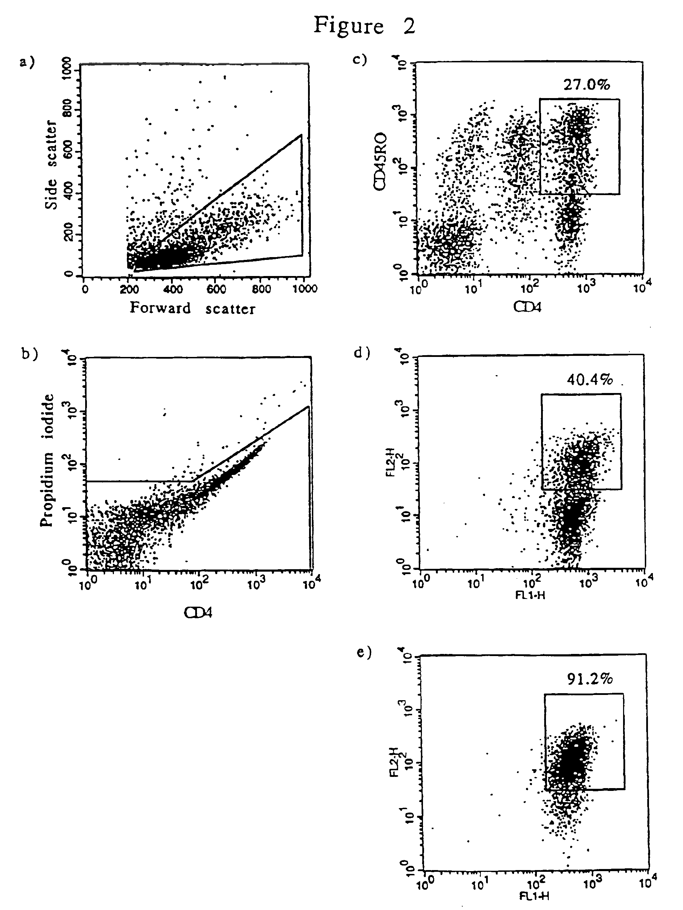

Figure 2 shows an analysis of a magnetic enrichment of CD45RO expressing T helper cells from PBMC. Figure 2a shows the forward-side scatter gating used for analysis. Figure 2b shows the PI versus CD45RO gating used for analysis. Figure 2c shows a Dot Plot of CD4 (FI-1) versus CD45RO (FI-2) of the cells before separation. Figure 2d shows a Dot Plot of CD4 (FI-1) versus CD45RO (FI-2) of the magnetic fraction after CD4 enrichment. Figure 2e shows a Dot Plot of CD4 (FI-1) versus CD45RO (FI-2) of the magnetic fraction after CD45RA isolation.

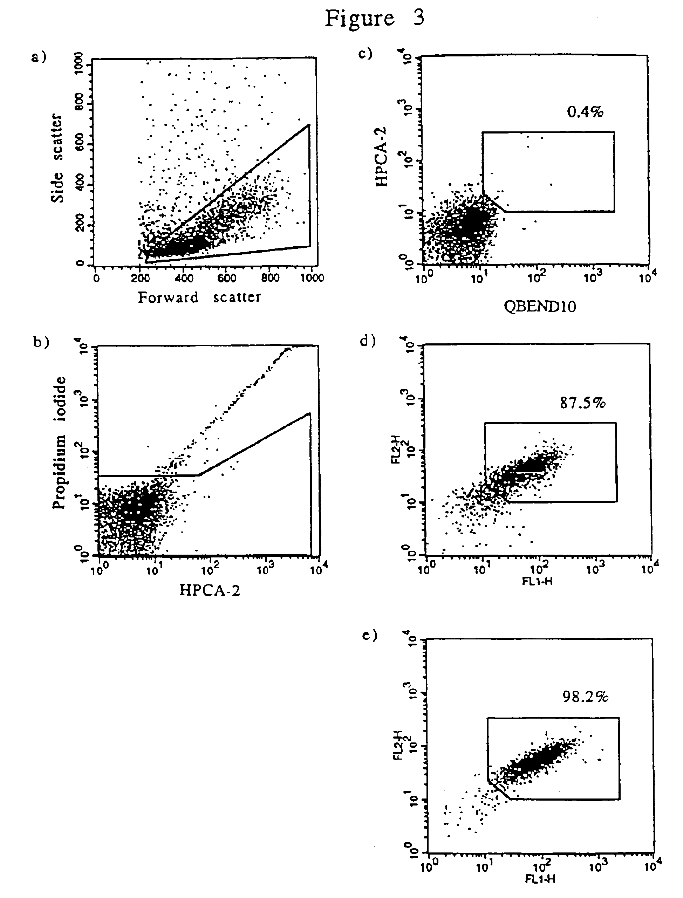

Figure 3 shows an analysis of a magnetic enrichment of CD34⁺ cells from PBMC with antibodies against two different CD34 epitopes. Figure 3a shows the forward-side scatter gating used for analysis. Figure 3b shows the PI versus HPCA2 gating used for analysis. Figure 3c shows a Dot Plot of QBEnd 10-FITC (FI-1) versus HPCA2-dig mAB plus α-dig-PE (FI-2) of the cells before separation. Figure 3d shows a Dot Plot of QBEnd 10-FITC (FI-1) versus HPCA2-dig mAB plus α-dig-PE (FI-2) of the magnetic fraction after QBEnd10 enrichment. Figure 3e shows a Dot Plot of QBEnd 10-FITC (FI-1) versus HPCA2-dig mAB plus α-dig-PE (FI-2) of the magnetic fraction after HPCA2 isolation.

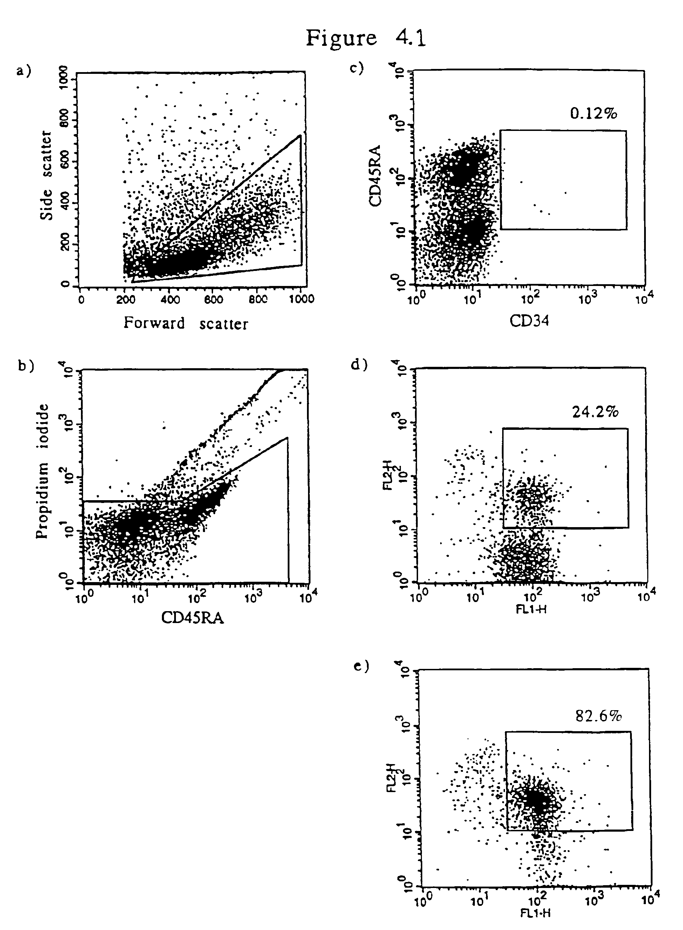

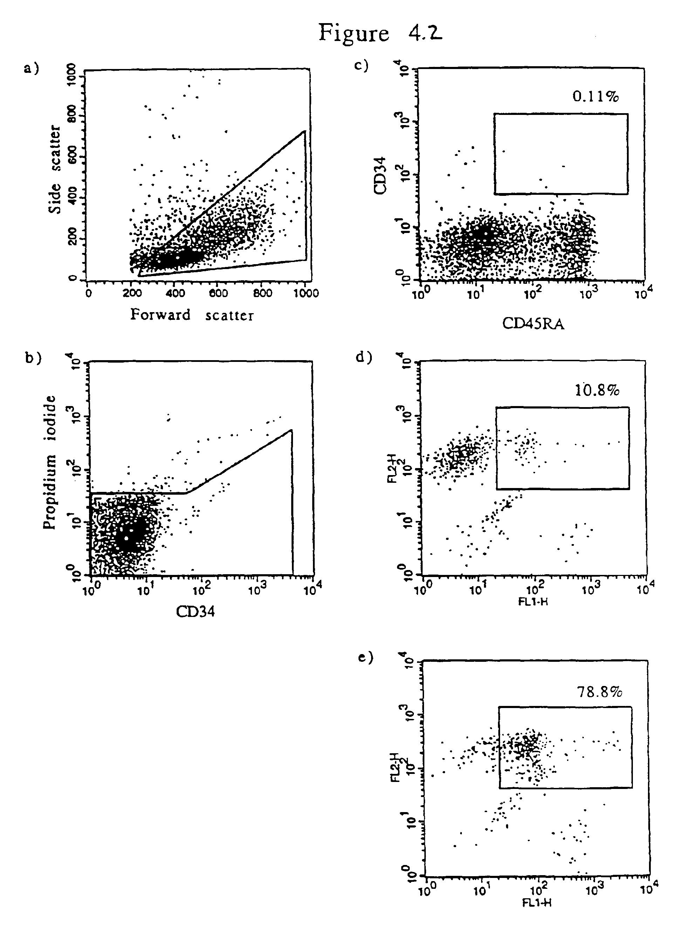

Figure 4.1 shows an analysis of a magnetic isolation of CD45RA expressing hematopoietic progenitor cells from PBMC. Figure 4.1a shows the forward-side scatter gating used for analysis. Figure 4.1b shows the PI versus CD45RA gating used for analysis. Figure 4.1c shows a Dot Plot of CD34 (FI-1) versus CD45RA (FI-2) of the cells before separation. Figure 4.1d shows a Dot Plot of CD34 (FI-1) versus CD45RA (FI-2) of the magnetic fraction after CD34 enruchment. Figure 4.1e shows a Dot Plot of CD34 (FI-1) versus CD45RA (FI-2) of the magnetic fraction after CD45RA isolation.
Figure 4.2 shows an analysis of a magnetic enrichment of CD45RA expressing CD34+ cells from PBMC. Figure 4.2a shows the forward-side scatter gating used for analysis. Figure 4.2b shows the PI versus CD34 gating used for analysis. Figure 4.2c shows a Dot Plot of CD45RA (FI-1 ) versus CD34 (FI-2) of the cells before separation. Figure 4.2d shows a Dot Plot of CD45RA (FI-1 versus CD34 (FI-2) of the magnetic fraction after CD34 enrichment. Figure 4.2e shows a Dot Plot of CD45RA (FI-1) versus CD34 (FI-2) of the magnetic fraction after CD45RA isolation.

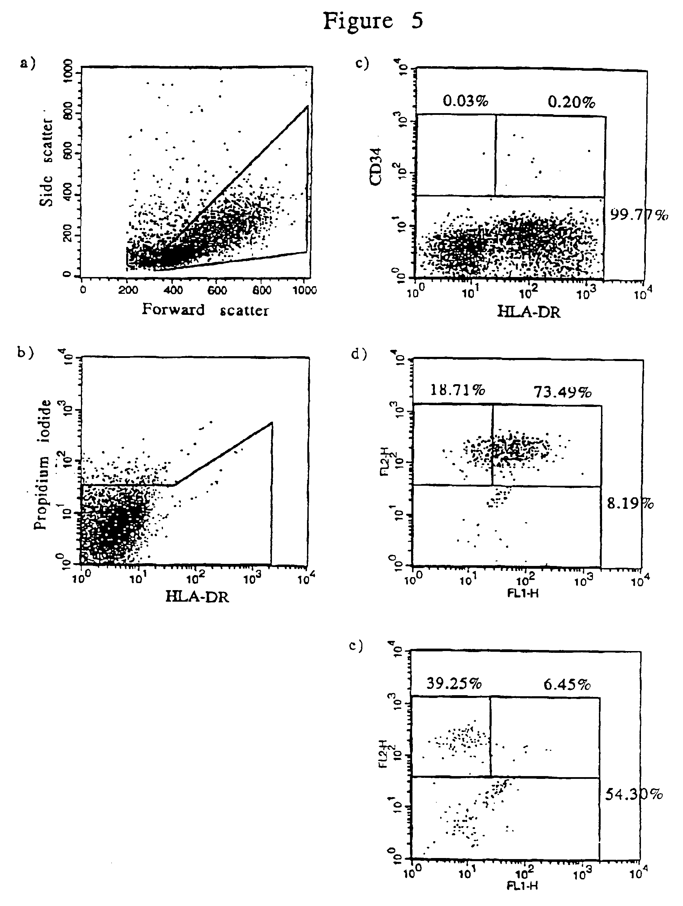

Figure 5 shows an analysis of a magnetic enrichment of HLA-DR negative CD34+ cells from PBMC. Figure 5a shows the forward and side scatter gating used for analysis. Figure 5b shows the PI versus CD34 gating used for analysis. Figure 5c shows a Dot Plot of HLA-DR (FI-1) versus CD34 (FI-2) of the cells before separation. Figure 5d shows a Dot Plot of HLA-DR (FI-1) versus CD34 (FI-2) of the magnetic fraction after CD34 enrichment. Figure 5e shows a Dot Plot of HLA-DR (FI-1) versus CD34 (FI-2) of the magnetic fraction after HLA-DR depletion.

### DESCRIPTION OF THE SPECIFIC EMBODIMENTS

Colloidal superparamagnetic particles (magnetic microparticles), attached to a cell by chemical linkage to an affinity reagent, are released by the activity of a glycosidase specific for a glycosidic linkage present either in the particle coating, or in the linkage group between the coating and the affinity reagent. The release process is used in cell separations where removal of magnetic particle labels is desirable.

Superparamagnetic particles are prepared with a coating, preferably a polysaccharide coating, having a glycosidic linkage that is absent in normal mammalian cells. The glycosidic linkage may be present in the coating itself, or may be introduced in a linker attaching the affinity reagent to the coating. Glycosidic linkages of interest will be absent on the surface of normal mammalian cells. After an initial separation step using the microparticles, the microparticles are released by the action of a glycosidase specific for the polysaccharide coating.

Suitable magnetic particles are described in Molday U.S. 4,452,773 and European Patent Specification 452342-B (published November 20, 1994). Colloidal sized particles, such as those described in Owen U.S. 4,795,698 and Liberti *et al.* U.S. 5,200,084, are also suitable. The microparticles will usually be less than about 100 nm in diameter, and usually will be greater than about 10 nm in diameter.

In preparation of the particles, a macromolecular coating will be used. Coatings of interest include dextrans, cellulose, amylose, pectin, chitin, etc., which have a convenient specific hydrolase useful as a releasing agent. The coating is suitably derivatized to provide functional groups for attachment of the affinity reagent. A variety of such modifications is known in the art. The functional groups are then used to link the microparticles to affinity reagents specific for markers present on a biological target of interest.

Amino groups may be introduced before or after forming the beads. An aldehyde function may be introduced by reacting a polysaccharide with diimidazol or DCCD, and coupling hexane diamine to the sugar molecules. Alternatively, in preparing dextran for coating, aminodextran may be mixed with unsubstituted dextran to provide amino groups. Preferably the functional, amino groups are introduced onto the ends of the polymer, *e.g*. on the 6-terminal sugar of the dextran molecule. The polysaccharides may be conveniently oxidized using periodate to provide aldehyde functional groups which can be conjugated to amino substituents on a proteinaceous binding moiety, particularly under the conditions of reductive amination. Antibodies can be coupled to the particles through side chain amino or sufhydryl groups and heterofunctional cross-linking reagents. In some cases the glycosidic side chain of the antibody can be oxidized by periodate and coupled with free amino groups of the molecules used for coating.

A large number of heterofunctional compounds are available for linking to entities. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-γ-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, NHS-PEG-MAL, Shearwater polymers, and succinimidyl 4-[N-maleimidomethyl]cyclohexane-1-carboxylate. A preferred linking group is 3-(2-pyridyldithio)propionic acid N-hydroxysuccinimide ester (SPDP) or 4-(N-maleimidomethyl)-cyclohexane-1-carboxylic acid N-hydroxysuccinimide ester (SMCC) with a reactive sutfhydryl group on the antibody and a reactive amino group on the magnetic particle.

The hydrolase, or releasing agent, will cleave a glycosidic linkage present on the macromolecular microparticle coating, or present in the linker. The releasing agent will leave substantially intact the surface antigens present on the biological target, *e.g*. cell, organelle, *etc.* Preferred release agents are enzymes that are reactive under physiological conditions, so as to minimize the effect on the target. After the release reaction, the viability of the cell is maintained, as is its ability to bind additional affinity reagents to cell surface markers, respond to agents through cell surface receptors, *etc*.

The choice of release agent will be determined by the glycosidic linkage. Where the macromolecular coating is a polysaccharide, the polysaccharide will be chosen to have glycosidic linkages not normally found in mammalian cells. Hydrolases that recognize specific glycosidic structures may be used as a release agent *e.g*. dextran and dextranase, which cleaves at the α(1→6) linkage; cellulose and cellulase, which cleaves at the β(1→4) linkage; amylose and amylase; pectin and pectinase; chitin and chitinase, *etc*.

The release agent is added to a cell population to which magnetic microparticles are bound through specific affinity reagents. The medium in which the cells are released will be any medium that maintains the viability of the cells and allows activity of the release agent. Suitable media include phosphate buffered saline containing from 0.1 to 0.5% BSA, Dulbecco's Modified Eagle Medium (dMEM), Hank's Basic Salt Solution (HBSS), Dulbecco's phosphate buffered saline (dPBS), RPMI, lscove's medium, PBS with 5 mM EDTA, etc., frequently supplemented with fetal calf serum, BSA, HSA, *etc*.

The amount of releasing agent added will be sufficient to release substantially all of the microparticles from the cells in the desired period of time. Usually at least about 80% of the microparticles, more usually at least about 95%, preferably at least about 99% of the microparticles are released. The conditions for release may be empirically optimized in terms of temperature, pH, presence of metal cofactors, reducing agents, *etc*. by varying such conditions and determining the quantitative effect on microparticle release. The release will usually be complete in at least about 15 minutes, more usually at least about 10 minutes, and will usually not be longer than about 30 minutes. The cells may be washed after the reaction is complete.

In order to prevent reaction of residual release agent with the microparticles during the following separation steps, blocking solution is usually added. The composition of the blocking solution will be selected based on the activity of the release agent, and will usually comprise a substrate for the enzyme, *e.g*. dextran for dextranases, cellulose for cellulases, etc., in an amount sufficient to competitively inhibit substantially all of the residual enzymatic activity toward the microparticles. Alternatively, the microparticles in the following steps may be chosen to have a linkage that is not susceptible to the first release agent, for example, iron starch particles in combination with a dextranase releasing agent.

As an illustration of a suitable release reaction, cells bound to microparticles coated with dextran are combined with dextranase, as described in the examples. The mixture is incubated at ambient temperature for about 1 to 5 minutes. The cells are washed, and resuspended. Optionally, dextran is added to the cell suspension, usually about 1 to 100 mg/ml, to prevent any further release of microparticles.

Specific cell subsets in a complex mixture are separated by high gradient magnetic separation on the basis of surface expression of specific markers. The separation process may be a depletion, where the desired subset is not magnetically labeled, or an enrichment, where the desired subset will be retained on a magnetic matrix. The subject release method allows rapid release of the microparticles without release of the affinity reagent, and without substantially altering the surface antigens present on the cell.

The release method is useful during purification of specific cell types from complex mixtures, where the procedure has multiple separation steps. The procedure may use a variety of combinations of enrichment and depletion steps, generally starting with an enrichment. In order to achieve a high degree of purification for a single marker, two sequential enrichment steps may be performed, using antibodies specific for two different epitopes of the marker, for example, two different anti-CD34 antibodies. Two sequential positive selections may be performed for different markers, for example, combining separations for two or more of CD34, thy-1, CD71, transferrin receptor, HB-F fetal cell selection, a cocktail of hematopoietic lineage markers, such as a combination of CD4/CD8/CD19, cytokine receptors, CD45RA/RO, *etc.* It is often desirable to first perform an enrichment when sorting a minor cell population from a complex mixture, in order to reduce the number of cells being manipulated. For example, an enrichment for CD34 positive cells during selection for hematopioetic progenitor cells, or an enrichment for CD71 positive cells during selection for fetal cells in maternal blood, are useful first steps. This enrichment is then followed by a depletion step, and optionally, another positive selection. The number of selection steps can be extended as necessary.

Especially useful affinity reagents are antibodies specific for cell surface antigens. Whole antibodies may be used, or fragments, *e.g*., Fab, F(ab')₂, light or heavy chain fragments, etc. Such antibodies may be polyclonal or monoclonal and are generally commercially available or alternatively, readily produced by techniques known to those skilled in the art. The antibodies may be directly or indirectly coupled to the microparticles. Indirect coupling can be accomplished by several methods. The antibodies may be coupled to one member of a high affinity binding system, *e.g*. biotin, and the particles attached to the other member, *e.g*. avidin. One may also use second stage antibodies that recognize species-specific epitopes of the depletion antibodies, *e.g.* anti-mouse Ig, anti-rat Ig, *etc.* Indirect coupling methods allow the use of a single magnetically coupled antibody species with a variety of depletion antibodies.

The antibodies are added to a cell suspension, and incubated for a period of time sufficient to bind the available cell surface antigens. The incubation will usually be at least about 1 minute and usually less than about 30 minutes. It is desirable to have a sufficient concentration of antibodies in the reaction mixture, such that the efficiency of the magnetic separation is not limited by lack of antibody. The appropriate concentration is determined by titration.

Where a second stage magnetically coupled antibody is used, the cell suspension may be washed and resuspended in medium as described above prior to incubation with the second stage antibodies. Alternatively, the second stage antibody may be added directly into the reaction mix. When directly coupled depletion antibodies are used, the cell suspension may be used directly in the next step, or washed and resuspended in medium.

The suspension of magnetically labeled cells is applied to a column or chamber as described in WO 90/07380. The matrix may consist of closely packed ferromagnetic spheres, steel wool, wires, magnetically responsive fine particles, etc. The matrix is composed of a ferromagnetic material, *e.g*. iron, steel, etc. and may be coated with an impermeable coating to prevent the contact of cells with metal. The matrix should have adequate surface area to create sufficient magnetic field gradients in the separation chamber to permit efficient retention of magnetically labeled cells. The volume necessary for a given separation may be empirically determined, and will vary with the cell size, antigen density on the cell surface, cell number, antibody affinity, *etc*.

The labeled cells are bound to the matrix in the presence of a magnetic field, usually at least about 100 mT, more usually at about 500 mT, usually not more than about 2T, more usually not more than about 1T. The source of the magnetic field may be a permanent or electromagnet. The unlabeled cells are washed from the column. The remaining cells are enriched for the subset which is positive for the desired marker.

The bound cell population may be released from the matrix by either (1) removing the magnetic field, or by (2) adding release agent to free the cells from the microparticles, using the previously described conditions. In the first case, the eluted cells are collected in a physiologically acceptable medium, and then treated with release agent, where the release step is performed as previously described. The released cells may be washed, and optionally treated with blocking solution.

The enriched cell population is then used in a second separation step. If an enrichment is performed, the separation is performed essentially as described for the first step, although with fewer cells. After binding to the matrix, the bound cells are released by removing the magnetic field, and eluting in a suitable buffer. The cells may be collected in any appropriate medium that maintains the viability of the cells. If the second separation step is a depletion, then the unbound cells are collected after passing the sample over the second magnetic matrix. The cells may be washed and concentrated after collection if desired. The procedure may link additional separation steps as required. After the separation is complete, the cells are used as appropriate. The method of harvesting will depend on the type of analysis to be performed.

In many cases analysis will be performed on aliquots of the original and separated populations to follow the separation procedure. It may be useful to label the antibodies with a fluorochrome, e.g. phycoerythrin, FITC, rhodamine, Texas red, allophycocyanin, *etc.* The fluorochrome label may be used to monitor microscopically or by flow cytometry the cell composition after the separation steps. Fluorescent labeling may conveniently utilize the same indirect coupling system as the magnetic particles.

In order to address the needs of research and clinical laboratories, a kit may be provided having the reagents and apparatus necessary to perform the subject invention. Such a kit will contain the release agent and microparticles having the appropriate coating or linker. A suitable blocking solution may be included. The microparticles may be provided already conjugated to affinity reagents, e.g. antibodies specific for cell markers, anti-hapten antibodies, anti-Ig antibodies, etc. When the microparticles are conjugated to second stage antibodies, suitable first stage antibodies may also be provided. Other components provided may be column(s), buffers for enzyme digestion, *etc.* While single columns may be used, it is anticipated that multiple columns will be run simultaneously, and an apparatus for automated or manual procedures may optionally be provided for such a purpose.

The following examples are offered by way of illustration and not by way of limitation.

### EXPERIMENTAL

### Materials and Methods

*Dextranase.* Dextranase from a selected strain of *Penicilium sp.* (Sigma, St. Louis, Missouri) and from *Penicilium liacinum* was used in the experiments. Standard purification methods were used to purify the enzyme. A purification procedure for dextranse from *Streptococccus sobrinus* UAB66 is described in Wanda and Curtiss (1994) J. Bacteriol. **176**:3839-3850. The preparations had a typical activity of 100-200 units/mg of protein. One unit will liberate 1.0 µmole of isomaltose per min at pH 6 at 37°C, using dextran as substrate.

*Direct Conjugation of antibodies to colloidal Iron Dextran particles.* 3 ml of amino modified colloidal sized Iron Dextran microbeads (Amino-Microbeads, Miltenyi Biotec, Germany) are activated with a solution of 3 mg SPDP (Pierce) (10 mg/ml in DMF) and reacted for 60 minutes at room temperature. The activated beads are purified through a type AS magnetic column (Miltenyi Biotec, Germany), and eluted in 3 ml of PBS-EDTA. 500 µg monoclonal antibody is reacted with iminothiolane in DMSO under conditions where 1-3 SH groups per antibody are introduced. Excess iminothiolane is removed by dialysis. The SH modified protein is added to the activated magnetic particles at a concentration of 10 to 40 µg per OD₄₅₀=10 and reacted for 2 hours at room temperature. The resulting conjugate is purified on a type AS high gradient magnetic column, and eluted in 10 ml of PBS-EDTA. Azide at 0.05% by weight is added as an antimicrobial agent.

*Conjugate of antibodies to colloidal Iron Dextran particles via additional Dextran Spacer (Spacer Microbeads).* Amino-Microbeads (Miltenyi Biotec, Germany) are activated by addition of 1 mg N-succinimidyl 3-(2-pyridyldithio)propionate (SPDP; Pierce) per ml beads for 60 minutes at room temperature (conjugation according to the specifications of the supplier). Excess SPDP is removed by passage over an AS magnetic separation column. The activated particles are coupled to SH modified 500 kD aminodextran (Molecular Probes, Oregon, approx. 0.5-SH group per dextran molecule). Antibodies can be coupled to the amino groups of the conjugated dextran molecules as described above.

*Preparation of human PBMC.* PBMC were obtained from leukocyte-rich buffy coats by centrifugation over Ficoll-Paque (Pharmacia, Uppsala, Sweden). After centrifugation, interphase cells were collected, resuspended in buffer and sedimented at 300 x g and then once again resuspended in buffer and centrifuged at 200 x g to remove platelets.

*Labeling of cells with CD34-Spacer-MicroBeads and CD34 PE for the first separation.* 10⁸ PBMC are incubated for 15 minutes at 8°C with CD34-Spacer Microbeads conjugated to anti-CD34 antibody (QBEnd10) and fluorochrome conjugated CD34 antibody (HPCA-2-PE, Becton Dickinson) for an additional 10 minutes then washed once with 10 ml PBS.

*Positive enrichment of CD34 positive cells by HGMS (MiniMACS sorting).* Magnetically labeled CD34⁺ cells were enriched on a MiniMACS MS column (magnetizable steelball matrix) inserted in a MiniMACS permanent magnet. To evaluate the efficiency of the cell separation, aliquots of unseparated cells and from the magnetic and non-magnetic cell fractions were analyzed by flow cytometry using a FACScan.

*Release of the colloidal supermagnetic particles from the enriched first marker*+ *cells.* The magnetic cell fraction was incubated with 20 µl of a 20 µg/ml dextranase solution per 1 ml cell suspension for 10 minutes at 8°C. The cells were passed over a MiniMACS separation column (Miltenyi Biotec) to remove cells with unreleased particles. The non-magnetic fraction is washed by centrifugation (300 x g). 20 µl of stop reagent (10% T40 Dextran (Pharmacia) in PBS) and PBS/BSA/EDTA were added to the cell pellet to a final volume of 100 µl.

*Magnetic and fluorescent labeling with CD45RA-conjugated colloidal superparamagnetic MicroBeads, CD45RA-PE and CD45RA-FLUOS.* The fluorescent staining of the non-magnetic fraction from the above separations was performed by addition of CD45RA-conjugated colloidal superparamagnetic microbeads (L48, Becton Dickinson) and incubation for 15 minutes at 8°C. The fluorescent staining for CD45 was done by subsequent incubation with CD45RA-FLUOS (L48, Becton Dickinson) for 10 minutes at 8°C or with CD45RA-PE (L48, Becton Dickinson) for 10 minutes at 8°C or with CD45RA-PE (clone: L48, Becton Dickinson) for 5 minutes at 8°C.

*Magnetic and fluorescent labeling with CD45RO-conjugated colloidal superparamagnetic MicroBeads and CD45RO-PE.* The fluorescent staining of the non-magnetic fraction from the above separations was performed by addition of CD45RO-conjugated colloidal superparamagnetic microbeads (UCHL1, CLB) and incubation for 15 minutes at 8°C. The fluorescent staining for CD45 was done by subsequent incubation with CD45RO-PE (UCHL1, CLB) for 5 minutes at 8°C.

*Magnetic and fluorescent labeling with CD4-FITC*_{*Isomer 1*} *and αFITC*_{*Isomer 1*}*-conjugated colloidal superparamagnetic MicroBeads.* The fluorescent staining of the non-magnetic fraction from the above separations was performed by addition of CD4-FITC_{Isomer 1} (Leu3a, Becton Dickinson) and incubation for 7 minutes at 8°C. The magnetic labeling was done by subsequent incubation with α FITC_{Isomer 1}-conjugated colloidal superparamagnetic microbeads (2f3.1, Samuel, UK; Harmer and Samuel (1989) J. Immunonol...Methods **122**:115-121) for 15 minutes at 8°C.

*Magnetic and fluorescent labeling with CD34-FITC*_{*Isomer 1*} *and αFITC*_{*Isomer 1*}*-conjugated colloidal superparamagnetic MicroBeads.* The fluorescent staining of the non-magnetic fraction from the above separations was performed by addition of CD34-FITC_{Isomer 1} (QBEnd 10, P. Grimsley, *et al.* (1993) Leukemia **7**:898-908) and incubation for 7 minutes at 8°C. The magnetic labeling is done by subsequent incubation with αFITC_{isomer 1⁻} conjugated colloidal superparamagnetic microbeads (2f3.1, Samuel, *supra.*) for 5 minutes at 8°C.

*Magnetic labeling with CD34-digoxigenin (dig) and α-dig-conjugated colloidal superparamagnetic MicroBeads.* The fluorescent staining of the non-magnetic fraction from the above separations was performed by addition of CD34-dig (HPCA2, Becton Dickinson) and incubation for 7 minutes at 8°C. The magnetic labeling was done by subsequent incubation with α-dig-conjugated colloidal superparamagnetic microbeads (Boehringer, Mannheim) for 15 minutes at 8°C.

*Positive enrichment of double-positive cells by HGMS (MiniMACS sorting).* Enrichment was performed as described above for CD34 positive cells.

*Magnetic and fluorescent labeling with HLA-DR-conjugated colloidal superparamagnetic MicroBeads and HLA-DR-FLUOS.* The fluorescent staining of the non-magnetic fraction from the above separations was performed by addition of 8 µl of HLA-DR-conjugated colloidal superparamagnetic microbeads (CLB HLA-DR) and incubation for 15 minutes at 8°C. The fluorescent staining is done by subsequent incubation with HLA-DR-FLUOS (910/D7 [BMA022] Behring) for 10 minutes at 8°C.

### Results

*Magnetic separation of CD45RA expressing T helper cells from PBMC.* 5 x 10⁷ PBMC in 500 µl PBS/EDTA/BSA were labeled for 15 minutes at 8°C with CD4-FITC (Becton Dickinson) (concentration according to the specifications of the supplier) then washed with PBS. After centrifugation, the cells were resuspended in 400 µl PBS and 100 µl magnetic microparticles prepared as described above, and conjugated to anti-FITC antibody (Harmer and Samuel (1989) J. Immunol...Methods **122**:115-121) were added. The cells were incubated for 15 minutes at 8°C, then washed once and resuspended in 1 ml PBS.

The cells were separated over a MiniMACS separation column (MS⁺). The non-magnetic and magnetic fractions were collected. The magnetic positive fraction, containing CD4⁺ T helper cells, was incubated with 20 µl dextranase solution to release the magnetic microparticles. These cells were separated over a MiniMACS separation column (MS⁺) to remove the cells that remained magnetically labeled. The cells of the non-magnetic fraction were resuspended after centrifugation in PBS to a final volume of 50 µl, and 30 µl Stop Reagent was added. This cell suspension was incubated with magnetic microparticles (OD₄₅₀=10, 1:200) prepared as described above, and conjugated to CD45RA-antibody (L48, Becton Dickinson). The fluorescent staining was done by subsequent incubation with PE conjugated CD45RA antibody (Becton Dickinson).

For analysis by flow cytometry, 1 µl (1 mg/ml) of propidium iodide was added to the sample and the cells were analyzed on a FACScan Flow Cytometer (Becton Dickinson). Debris and dead cells were excluded by gating.

Figure 1a shows the forward-side scatter gating used for analysis. Figure 1b shows the FI-1 versus FL-3 gating used for analysis. Figure 1c shows a Dot Plot of CD4 (FI-1) versus CD45RA (FI-2) of the cells before separation, 22.5% of the cells express CD4 and CD45RA. Figure 1d shows a Dot Plot of CD4 (FI-1) versus CD45RA (FI-2) of the magnetic fraction after CD4 separation, 54.4% of the cells express CD4 and CD45RA. Figure 1e shows a Dot Plot of CD4 (FI-1) versus CD45RA (FI-2) of the magnetic fraction after CD45RA separation, >96.6% of the cells express CD4 and CD45RA.

*Magnetic separation of CD45RO expressing T helper cells from PBMC.* 5 x 10⁷ PBMC in 500 µl PBS/EDTA/BSA were labeled for 15 minutes at 8°C with CD4-FITC (Becton Dickinson, concentration according to the specifications of the supplier) then washed with PBS. After centrifugation, the cells were resuspended in 400 µl PBS and 100 µl magnetic microparticles prepared as described above, and conjugated to anti-FITC antibody (Harmer and Samule (1989) J. Immunol. Methods **122**:115-121) were added. The cells were incubated for 15 minutes at 8°C, then washed once and resuspended in 1 ml PBS.

The cells were separated over a MiniMACS separation column (MS⁺). The non-magnetic and magnetic fractions were collected. The magnetic positive fraction, containing CD4⁺ T helper cells, was incubated with 20 µl dextranase solution to release the magnetic microparticles. These cells were separated over a MiniMACS separation column (MS⁺) to remove the cells that remained magnetically labeled. The cells of the non-magnetic fraction were resuspended after centrifugation in PBS to a final volume of 50 µl, and 30 µl Stop Reagent was added. This cell suspension is incubated with magnetic microparticles (OD₄₅₀=10, 1:200) prepared as described above, and conjugated to CD45RO-antibody (UCHL1). The fluorescent staining was done by subsequent incubation with PE conjugated CD45RO antibody (Becton Dickinson).

For analysis by flow cytometry, 1 µl (1 mg/ml) of propidium iodide was added to the sample and the cells were analyzed on a FACScan Flow Cytometer (Becton Dickinson). Debris and dead cells were excluded by gating.

Figure 2a shows the forward-side scatter gating used for analysis. Figure 2b shows the FI-1 versus FL-3 gating used for analysis. Figure 2c shows a Dot Plot of CD4 (FI-1) versus CD45RO (FI-2) of the cells before separation, 27.0% of the cells express CD5 and CD45RO. Figure 2d shows a Dot Plot of CD4 (FI-1) versus CD45RO (FI-2) of the magnetic fraction after CD4 separation, 40.4% of the cells express CD4 and CD45RO. Figure 2e shows a Dot Plot of CD4 (FI-1) versus CD45RO (FI-2) of the magnetic fraction after CD45RA separation, more than 91.2% of the cells express CD4 and CD45RO.

*Magnetic enrichment of CD34*^{*+*} *cells from PBMC with antibodies against two different CD34 epitopes.* This experiment shows that the purity of a cell preparation after separation can be further increased by a second separation with an antibody recognizing a different epitope.

1.5 x 10⁸ PBMC in 1 ml PBS/EDTA containing 4 mg/ml Beriglobin (Behringwereke Marburg, Germany) were labeled with anti-CD34-FITC (QBEnd 10, Grimsley *et al.* (1993) Leukemia **7**:898-908) and with digoxigenin conjugated anti-CD34 antibody (HPCA2, Becton Dickinson) for 15 minutes at 8°C then washed with PBS. After centrifugation, the cells were resuspended in 500 µl PBS and 500 µl magnetic microparticles (OD₄₅₀=10, 1:200) prepared as described above, and conjugated to anti-FITC antibody (Harmer and Samule (1989) J. Immunol. Methods **122**:115-121) were added. The cells were incubated for 15 minutes at 8°C, then washed once and resuspended in 1 ml PBS.

The cells were separated over two MiniMACS separation column (MS⁺) to obtain optimal purity of the positively selected cells. The magnetic positive fraction, containing QBEnd 10⁺ cells, was incubated with 20 µl dextranase solution to release the magnetic microparticles. These cells were then separated over a MiniMACS separation column (MS⁺) to remove the cells that remained magnetically labeled. The cells of the non-magnetic fraction were resuspended after centrifugation in PBS to a final volume of 50 µl, and 30 µl Stop Reagent was added. The magnetic labeling was done by addition of magnetic magnetic microparticles (OD₄₅₀=10, 1:200) prepared as described above, and conjugated to α-Digoxygenin-antibody (Boehringer Mannheim, Germany) for 8 minutes at 8°C and the fluorescent staining by subsequent incubation with PE conjugated α-Digoxygenin-antibody (Boehringer Mannheim, Germany) for 8 minutes at 8°C.

For analysis by flow cytometry, 1 µl (1 mg/ml) of propidium iodide was added to the sample and the cells were analyzed on a FACScan Flow Cytometer (Becton Dickinson). Debris and dead cells were excluded by gating. The total enrichment rate is 13,000 to 50,000 fold.

Figure 3a shows the forward-side scatter gating used for analysis. Figure 3b shows the FI-2 versus FL-3 gating used for analysis. Figure 3c shows a Dot Plot of QBEnd 10-FITC (FI-1) versus HPCA2-dig mAB plus α-dig-PE (FI-2) of the cells before separation. Less than 0.4% of the total cells express CD34. Figure 3d shows a Dot Plot of QBEnd 10-FITC (FI-1) versus HPCA2-dig mAB plus α-dig-PE (FI-2) of the magnetic fraction after CD4 separation. The purity of CD34 cells was 87.5%. Figure 3e shows a Dot Plot of QBEnd 10-FITC (FI-1) versus HPCA2-dig mAB plus α-dig-PE (FI-2) of the magnetic fraction after CF45RA separation. The purity of CD34 cells is better than 98.2%

*Magnetic separation of CD45RA expressing hematopoietic progenitor cells from PBMC.* 3 x 10⁸ PBMC in 1.5 ml PBS/EDTA/BSA were labeled for 15 minutes at 8°C with α-CD34-FITC (QBEnd 10, Grimsley *et al.* (1993) Leukemia 7:898-908) then washed with PBS. After centrifugation the cells were resuspended in 750 µl PBS and 750 µl magnetic microparticles (OD₄₅₀=10, 1:200) prepared as described above, and conjugated to anti-FITC antibody (Harmer and Samule (1989) J. Immunol. Methods **122:**115-121) were added. The cells were incubated for 15 minutes at 8°C, then washed once and resuspended in 1 ml PBS.

The cells were separate by two runs over two MiniMACS separation column (MS⁺). The magnetic positive fraction, containing CD34⁺ cells, was incubated with 20 µl dextranase solution to release the magnetic microparticles. These cells were separated over a MiniMACS separation column (MS+) to remove cells that remained magnetically labeled. The cells of the non-magnetic fraction were resuspended after centrifugation in PBS to a final volume of 50 µl, and 30 µl Stop Reagent was added. This cell suspension was incubated with magnetic microparticles (OD₄₅₀=10, 1:200) prepared as described above, and conjugated to CD45RA-antibody (L48, Becton Dickinson). The fluorescent staining was done by subsequent incubation with PE conjugated CD45RA antibody (L48, Becton Dickinson.

For analysis by flow cytometry, 1 µl (1 mg/ml) of propidium iodide was added to the sample and the cells were analyzed on a FACScan Flow Cytometer (Becton Dickinson). Debris and dead cells were excluded by gating.

Figure 4.1 a shows the forward-side scatter gating used for analysis. Figure 4.1b shows the FI-2 versus FL-3 gating used for analysis. Figure 4.1 c shows a Dot Plot of CD34 (FI-1) versus CD45RA (FI-2) of the cells before separation, less than 0.12% of the cells express CD34 and CD45RA. Figure 4.1d shows a Dot Plot of CD34 (FI-1) versus CD45RA (FI-2) of the magnetic fraction after CD4 separation, 24.2% of the cells express CD34 and CD45RA. Figure 4.1 e shows a Dot Plot of CD34 (FI-1) versus CD45RA (FI-2) of the magnetic fraction after CD45RA separation.
More than 82.6% of the cells express CD34 and CD45RA

*Magnetic separation of CD45RA expressing CD34*+ *cells from PBMC.* 10⁸ PBMC in 500 µl PBS/EDTA/BSA were labeled for 15 minutes at 8°C with magnetic microparticles (OD₄₅₀=10, 1:200) prepared as described above, and conjugated to α-CD34 antibody-FITC (QBEnd 10, P. Grimsley *et al.* (1993) Leukemia **7**:898-908) then washed with PBS. The fluorescent staining was done by subsequent incubation with PE conjugated CD34 antibody (HPCA2, Becton Dickinson) for 10 minutes at 8°C, then washed once and resuspended in 1 ml PBS.

The cells were separated over two MiniMACS separation column (MS⁺). The non-magnetic and magnetic fractions were collected. The magnetic positive fraction, containing CD34⁺ cells, was incubated with 20 µl dextranase solution to release the magnetic microparticles. These cells were separated over a MiniMACS separation column (MS⁺) to remove the cells that remained magnetically labeled. The cells of the non-magnetic fraction were resuspended after centrifugation in PBS to a final volume of 50 µl and 30 µl Stop Reagent is added. This cell suspension is incubated with magnetic microparticles (OD₄₅₀=10, 1:200) prepared as described above, and conjugated to CD45RA-antibody (L48, Becton Dickinson). The fluorescent staining is done by subsequent incubation with FLUOS conjugated CD45RA antibody (L48, Becton Dickinson).

For analysis by flow cytometry, 1 µl (1 mg/ml) of propidium iodide was added to the sample and the cells were analyzed on a FACScan Flow Cytometer (Becton Dickinson). Debris and dead cells were excluded by gating.

Figure 4.2a shows the forward-side scatter gating used for analysis. Figure 4.2b shows the FI-2 versus FL-3 gating used for analysis. Figure 4.2c shows a Dot Plot of CD45RA (FI-1) versus CD34 (FI-2) of the cells before separation, <0.11% of the cells express CD34 and CD45RA. Figure 4.2d shows a Dot Plot of CD45RA (FI-1 versus CD34 (FI-2) of the magnetic fraction after CD34 separation, 10.8% of the cells express CD34 and CD45RA. Figure 4.2e shows a Dot Plot of CD45RA (FI-1) versus CD34 (FI-2) of the magnetic fraction after CD45RA separation, 78.8% of the cells express CD34 and CD45RA.

*Magnetic enrichment of HLA-DR negative CD34*+ *cells from PBMC.* 10⁸ PBMC in 500 µl PBS/EDTA/BSA were labeled for 15 minutes at 8°C with magnetic microparticles (OD₄₅₀=10, 1:200) prepared as described above, and conjugated to CD34 antibody-FITC (QBEnd 10, P. Grimsley *et al.* (1993) Leukemia **7**:898-908) then washed with PBS. The fluorescent staining was done by subsequent incubation with PE conjugated CD34 antibody (HPCA2, Becton Dickinson) for 10 minutes at 8°C, then washed once and resuspended in 1 ml PBS.

The cells were separated over two MiniMACS separation columns (MS⁺). The non-magnetic and magnetic fractions were collected. The magnetic positive fraction, containing CD34⁺ cells, was incubated with 20 µl dextranase solution to release the magnetic microparticles. These cells were separated over a MiniMACS separation column (MS⁺) to remove cells that remained magnetically labeled. The cells of the non-magnetic fraction were resuspended after centrifugation in PBS to a final volume of 50 µl, and 30 µl Stop Reagent was added. This cell suspension was incubated for 15 minutes at 8°C with magnetic microparticles (OD₄₅₀=10, 1:200) prepared as described above, and conjugated to α-HLA-DR antibody (CLB, the Netherlands). The fluorescent staining was done by subsequent incubation for 10 minutes at 8°C with FLUOS conjugated HLA-DR antibody (910/D7 (BMA022, Behring, Germany)).

For analysis by flow cytometry, 1 µl (1 mg/ml) of propidium iodide was added to the sample and the cells were analyzed on a FACScan Flow Cytometer (Becton Dickinson). Debris and dead cells were excluded by gating.

Figure 5a shows the forward and side scatter gating used for analysis. Figure 5b shows the FI-1 versus FL-3 gating used for analysis. Figure 5c shows a Dot Plot of HLA-DR (FI-1) versus CD34 (FI-2) of the cells before separation, 0.2% of the CD34 cells are positive for HLA-DR. Figure 5d shows a Dot Plot of HLA-DR (FI-1) versus CD34 (FI-2) of the magnetic fraction after CD34 separation, 20.3% of the cells express CD34 and CD45RA. Figure 5e shows a Dot Plot of HLA-DR (FI-1) versus CD34 (FI-2) of the magnetic fraction after CD45RA separation, >39% of the cells express CD34 and CD45RA, 6.45% of the cells are positive for HLA-DR.

It is evident from the above results that the subject invention provides for a simple, fast method for performing multiple magnetic separations. Multiple separations can provide increased purity of the separated populations, by utilizing antibodies directed against multiple epitopes of a single marker. Multiple separations also allow complex separations, where positive selection steps and negative selections steps are combined. The ease of operation, and ability to scale up the number and size of samples, provide significant benefits over existing methods.

## Claims

1. A method for releasing cells bound through an affinity reagent to superparamagnetic microparticles, the method comprising:
combining said cells with a glycosidase specific for a glycosidic linkage present in at least one of (a) the coating of said microparticles and (b) a linkage group between said coating and said affinity reagent, wherein said glycosidic linkage is absent from normal mammalian cells; and,
incubating said cells with said glycosidase to release said microparticles from said cells.

2. A method according to claim 1, wherein said glycosidic linkage is an α (1-6) linkage present in a dextran coating on said microparticle, and said release agent is dextranase.

3. A method according to claim 2, wherein said affinity reagent is an antibody specific for a cell surface marker on said cells.

4. A method according to claim 3 wherein the cells are mammalian cells.

5. A method according to claim 4, wherein said cells are hematopoietic cells.

6. A method according to claim 5, wherein said antibody is specific for CD34 or CD71.

7. A method for separating a mammalian cell population subset from a mixture of cells using high gradient magnetic separation, the method comprising:
(a) performing a first selection process, comprising:
combining said mixture of cells with an antibody specific for a first cell surface antigen expressed by said cell population subset, coupled to a superparamagnetic microparticle coated with a polysaccharide having a glycosidic linkage absent from normal mammalian cells;
passing said mixture of cells through a ferromagnetic matrix, in the presence of a magnetic field, wherein cells bound by said antibody are retained, washing said matrix free of unbound cells; eluting said cell population subset from said matrix; combining said eluted cell population with a glycosidase capable of cleaving said glycosidic linkage; and,
incubating the eluted cell population with the glycosidase to release said cells; and
(b) performing a second selection process comprising:
combining said mixture of cells and an antibody specific for a second cell surface antigen or a second epitope of said first cell surface antigen expressed by said cell population subset, coupled to a superparamagnetic microparticle; passing said mixture of cells through a ferromagnetic matrix, in the presence of a magnetic field, wherein cells bound by said antibody are retained, and collecting at least one of said bound or unbound cell population;
wherein said cell population subset is enriched in relation to the original mixture.

## Patentansprüche

1. Verfahren zum Ablösen von Zellen, die durch ein Affinitätsreagens an superparamagnetische Mikropartikel gebunden sind, umfassend:
das Kombinieren der Zellen mit einer Glykosidase, die für eine glykosidische Bindung spezifisch ist, die in zumindest einer aus (a) der Beschichtung der Mikropartikel und (b) einer Bindungsgruppe zwischen der Beschichtung und dem Affinitätsreagens vorhanden ist, worin die glykosidische Bindung in normalen Säugetierzellen nicht vorhanden ist; und
das Inkubieren der Zellen mit der Glykosidase, um die Mikropartikel von den Zellen abzulösen.

2. Verfahren nach Anspruch 1, worin die glykosidische Bindung eine α-(1-6-)-Bindung ist, die in einer Dextranbeschichtung am Mikropartikel vorhanden ist, und das Ablösemittel Dextranase ist.

3. Verfahren nach Anspruch 2, worin das Affinitätsreagens ein Antikörper ist, der für einen Zelloberflächenmarker an den Zellen spezifisch ist.

4. Verfahren nach Anspruch 3, worin die Zellen Säugetierzellen sind.

5. Verfahren nach Anspruch 4, worin die Zellen hämatopoetische Zellen sind.

6. Verfahren nach Anspruch 5, worin der Antikörper für CD34 oder CD71 spezifisch ist.

7. Verfahren zur Abtrennung einer Säugetierzellpopulation-Untergruppe aus einem Gemisch von Zellen unter Verwendung von Hochgradienten-Magnetsortierung, umfassend:
(a) das Durchführen eines ersten Selektionsverfahrens, das:
das Kombinieren des Zellgemisches mit einem Antikörper, der für ein erstes Zelloberflächenantigen spezifisch ist, das von der Zellpopulation-Untergruppe exprimiert wird, gebunden an ein superparamagnetisches Mikropartikel, das mit einem Polysaccharid mit einer glykosidischen Bindung beschichtet ist, die in normalen Säugetierzellen nicht vorhanden ist;
das Hindurchführen des Zellgemisches durch eine ferromagnetische Matrix in Gegenwart eines Magnetfeldes, worin die von den Antikörpern gebundenen Zellen zurückgehalten werden, das Waschen der Matrix, um ungebundene Zellen zu entfernen; das Eluieren der Zellpopulation-Untergruppe aus der Matrix; das Kombinieren der eluierten Zellpopulation mit einer Glykosidase, die in der Lage ist, die glykosidische Bindung zu spalten; und
das Inkubieren der eluierten Zellpopulation mit der Glykosidase, um die Zellen abzulösen, umfasst; und
(b) das Durchführen eines zweiten Selektionsverfahrens, das:
das Kombinieren des Zellgemisches mit einem Antikörper, der für ein zweites Zelloberflächenantigen oder ein zweites Epitop von diesem ersten Zelloberflächenantigen spezifisch ist, das von der Zellpopulation-Untergruppe exprimiert wird, gebunden an ein superparamagnetisches Mikropartikel; das Hindurchführen des Zellgemisches durch eine ferromagnetische Matrix in Gegenwart eines Magnetfeldes, worin die an den Antikörper gebundenen Zellen zurückgehalten werden, und das Gewinnen von zumindest einer der gebundenen oder ungebundenen Zellpopulation umfasst;
worin die Zellpopulation-Untergruppe im Vergleich zum ursprünglichen Gemisch angereichert ist.

## Revendications

1. Méthode pour libérer des cellules liées par un réactif d'affinité à des microparticules superparamagnétiques, la méthode comprenant :
la combinaison desdites cellules avec une glycosidase spécifique d'un enchaînement glycosidique présent dans au moins l'un de (a) l'enrobage de sdites microparticules et (b) un groupe d'enchaînement entre ledit enrobage et ledit réactif d'affinité, où ledit enchaînement glycosique est absent de cellules mammaliennes normales ; et,
l'incubation desdites cellules avec ladite glycosidase pour libérer lesdites microparticules desdites cellules.

2. Méthode selon la revendication 1, où ledit enchaînement glycosidique est un enchaînement α (1 -6) présent dans un enrobage de dextrane sur ladite microparticule et ledit agent de libération est la dextranase.

3. Méthode selon la revendication 2, où ledit réactif d'affinité est un anticorps spécifique d'un marqueur de surface de cellules sur lesdites cellules.

4. Méthode selon la revendication 3, où lesdites cellules sont des cellules mammaliennes.

5. Méthode selon la revendication 4, où lesdites cellules sont des cellules hématopoïétiques.

6. Méthode selon la revendication 5, où ledit anticorps est spécifique de CD34 ou CD71.

7. Méthode pour séparer un sous-groupe d'une population de cellules mammaliennes d'un mélange de cellules en utilisant un e séparation magnétique à gradient élevé, la méthode consistant à :
(a) accomplir un premier processus de sélection comprenant :
combinaison dudit mélange de cellules avec un anticorps spécifique d'un premier antigène de surface de cellule exprimé par ledit sous groupe de population de cellule s couplé à une micro-particule superparamagnétique enrobée d'un polysaccharide ayant un enchaînement glycosidique absent des cellules mammaliennes normales ;
passage dudit mélange de cellules à travers une matrice ferromagnétique en présence d'un champ magnétique où les cellules liées par ledit anticorps sont retenues, lavage de ladite matrice pour la débarrasser des cellules non liées, élution dudit sous-groupe de population de cellules de ladite matrice ; combinaison de ladite population éluée de cellules avec une glycosidase capable de scinder ledit enchaînement glycosidique ; et incubation de la population de cellules éluées avec la glycosidase pour libérer lesdites cellules ; et
(b) accomplir un second processus de sélection comprenant :
combinaison dudit mélange de cellules et d'un anticorps spécifique d'un second antigène de surface de cellule ou un second épitope dudit premier antigène de surface de cellule exprimé par ledit sous-groupe de population de cellule s couplé à une particule superparamagnétique ; passage du dit mélange de cellules à travers une matrice ferromagnétique, en présence d'un champs magnétique, où les cellules liées par ledit anticorps sont re tenues, et collecte au moins de l'une de ladite population de cellules liées ou non liées ;
où ledit sous-groupe de population de cellule s est enrichi relativement au mélange d'origine.
